# FASCICULE DE BREVET EUROPEEN

(11) **EP 1 602 356 B1**
(45) Date de publication et mention de la délivrance du brevet: **11.03.2009**
(21) Numéro de dépôt: 05291111.2
(22) Date de dépôt: 24.05.2005
(51) Int. Cl.: A61K 8/81, A61K 8/23, A61K 8/37, A61K 8/44, A61Q 5/08

(54) **Compositions de décoloration de fibres kératiniques comprenant un composé polycarboxylique particulier, compositions prêtes à l'emploi, procédé de décoloration**
Mittel zum Entfärben von Keratinfasern enthaltend eine Polycarboxylverbindung, anwendungsfertiges Mittel und Bleichverfahren
Compositions for the decoloration of keratinic fibres comprising a polycarboxylic compound, compositions ready for use and process of decoloration

(30) Priorité: 28.05.2004 FR 0405836
(43) Date de publication de la demande: 07.12.2005
(73) Titulaire: L'ORÉAL, 75008 Paris (FR)
(72) Inventeur: Legrand, Frédéric, 92400 Courbevoie (FR)
(74) Mandataire: Fevrier, Murielle Françoise E.

(56) Documents cités:
- EP-A- 0 583 767
- EP-A- 1 380 287
- DE-A1- 19 909 661
- FR-A- 2 842 099
- FR-A- 2 842 101
- US-A1- 2002 141 954
- US-A1- 2003 106 564
- ED.,: R. C. PEPE, J.A. WENNINGER, G.N. MCEWEN: "INTERNATIONAL COSMETIC INGREDIENT DICTIONARY AND HANDBOOK" 2002, THE COSMETIC, TOILETRY AND FRAGRANCE ASSOCIATION , WASHINGTON, D.C. , XP002317650 9ème édition 2002 ISBN 1-882621-29-8 vol. 2, p. 1755-1756 * page 1755, colonne 3 - page 1756, colonne 1 *

## Description

La présente invention a pour objet une composition de décoloration de fibres kératiniques humaine comprenant au moins un composé portant quatre fonctions acide carboxylique, sous forme acide ou sous forme de sel, et une composition de décoloration prête à l'emploi susceptible d'être obtenue à partir de ladite composition et d'une composition comprenant au moins un agent oxydant. Elle concerne de plus un procédé de décoloration de fibres kératiniques appliquant ladite composition prête à l'emploi ainsi qu'un dispositif à plusieurs compartiments pour la mise en oeuvre de ce procédé.

Il est connu de décolorer des fibres kératiniques humaines, et en particulier les cheveux, en utilisant des compositions de décoloration comprenant un ou plusieurs agents oxydants. Parmi les agents oxydants classiquement utilisés, on peut citer le peroxyde d'hydrogène ou des composés susceptibles de produire du peroxyde d'hydrogène par hydrolyse, comme par exemple l'urée ou les persels tels que les perborates, les persulfates, les percarbonates.

A l'origine, les compositions de décoloration se présentaient sous la forme de poudre. Elles avaient cependant l'inconvénient de produire de la poussière durant leur manutention, leur transport et leur stockage. De plus, ce phénomène était aggravé par le fait que les produits qui composent ces poudres sont corrosifs, irritants pour les yeux, pour les voies respiratoires et les muqueuses.

C'est pourquoi, afin de résoudre les problèmes rencontrés avec l'usage de compositions pulvérulentes, on a récemment développé compositions de décoloration comprenant au moins un liquide inerte avec des teneurs permettant soit d'obtenir une composition sous forme de poudre dont la granulométrie est plus élevée que celle de la poudre initiale, soit d'obtenir une composition sous forme de pâte.

Cependant, si l'emploi de ce composé liquide inerte permet d'apporter une amélioration au problème causé par la volatilité de la poudre décolorante, il n'est pas toujours sans effet sur les performances de décoloration. En effet, le liquide inerte peut être la cause d'une certaine inhibition de la décoloration, ce qui peut se traduire par l'emploi de quantités plus importantes de composition, ou bien encore par un temps de pause plus important, avec en conséquence, une dégradation accrue des fibres kératiniques traitées.

La présente invention a pour objet de résoudre ces difficultés. Elle permet en effet d'accéder à des compositions de décoloration ne présentant pas de problème de toxicité due à la présence de très fines particules grâce à la présence d'un liquide inerte organique, sans perdre en efficacité de décoloration

Ainsi, elle a pour objet une composition de décoloration des fibres kératiniques humaines comprenant :
- au moins au moins un composé choisi parmi l'acide éthylènediamine-N,N'-disuccinique, un de ses sels, ou leur mélange.
- au moins un liquide inerte organique,
- au moins un persulfate.

Elle a de plus pour objet une composition de décoloration prête à l'emploi susceptible d'être obtenue par mélange de la composition précitée, avec une composition comprenant au moins un agent oxydant.

Un autre objet de l'invention concerne un procédé de décoloration de fibres kératiniques humaines, en particulier des cheveux, consistant à appliquer la composition prête à l'emploi sur les fibres kératiniques humaines à décolorer, sèches ou humides ; à la laisser agir pendant un temps de pause suffisant pour obtenir la décoloration recherchée ; à éliminer la composition par un rinçage à l'eau.

Enfin, l'invention concerne un dispositif à plusieurs compartiments, ou "kit", pour la mise en oeuvre du procédé de décoloration précité, comportant au moins deux compartiments dont l'un contient une composition sous forme de pâte ; et l'autre contient une composition comprenant au moins un agent oxydant.

Il a été trouvé que les compositions de décoloration prêtes à l'emploi selon l'invention s'appliquent facilement et rapidement. Elles présentent par ailleurs une très bonne adhérence sur les fibres et ne coulent pas hors des zones que l'on souhaite décolorer.

Elles permettent enfin d'obtenir des décolorations puissantes et homogènes, tout en offrant de très bonnes propriétés cosmétiques aux fibres ainsi traitées.

Mais d'autres caractéristiques et avantages de la présente invention apparaîtront plus clairement à la lecture de la description et des exemples qui vont suivre.

Dans ce qui va suivre et à moins que le contraire ne soit précisé, les bornes délimitant un domaine de valeurs sont comprises dans ce domaine.

Par ailleurs, les fibres kératiniques humaines traitées conformément à l'invention, sont plus particulièrement des cheveux.

Ainsi que cela a été indiqué auparavant, la composition selon l'invention comprend au moins un composé choisi parmi l'acide éthylènediamine-N,N'-disuccinique, un de ses sels, ou leur mélange.

Les sels sont de préférence des sels de métaux alcalins (comme par exemple le sodium, le potassium), de métaux alcalino-terreux (notamment le calcium, le magnésium), d'ammonium substitués ou non par un à trois radicaux alkyle, identiques ou différents, comprenant 1 à 6 atomes de carbone, de préférence de 1 à 4 atomes de carbone, éventuellement porteurs d'au moins un radical hydroxyle.

De préférence, le composé entrant dans la composition selon l'invention se présente sous la forme d'un sel de sodium.

Conformément à un mode de réalisation particulier de l'invention, la teneur en acide éthylènediamine-N,N'-disuccinique, sous forme acide ou sel, ou en mélange, représente entre 0,01 et 20 % en poids par rapport au poids de la composition, plus particulièrement entre 0,1 et 10 % en poids par rapport au poids de la composition, de préférence entre 0,2 et 5 % en poids par rapport au poids de la composition.

La composition selon l'invention comprend de plus au moins un liquide inerte organique.

Par liquide, on entend un composé ou un mélange de composés liquides à 25°C et à pression atmosphérique.

Avantageusement, le liquide inerte organique est choisi parmi les polydécènes, les mono- et poly- esters d'acides carboxyliques, les mono- ou poly- esters de sucres d'acides en C₈-C₃₀, les éthers cycliques, les esters cycliques, les huiles silicones, les huiles minérales, les huiles végétales, ou leurs mélanges.

Plus particulièrement, les polydécènes sont des composés de formule C₁₀ₙH_{[(20n)+2]} avec n variant de 3 à 9, et de préférence de 3 à 7. Ces composés répondent à l'appellation "polydécène" du Dictionnaire CTFA 7ème édition 1997 de la Cosmetic, Toiletry and Fragrance Association, USA, ainsi qu'à la même appellation INCI aux USA et en Europe. Ce sont des produits d'hydrogénation des poly-1-décènes.

On peut citer, à titre d'exemple, le produit vendu sous la dénomination Silkflo^{®} 366 NF Polydecene par la société Amoco Chemical, ceux vendus sous la dénomination Nexbase® 2002 FG, 2004 FG, 2006 FG et 2008 FG par la société Fortum.

En ce qui concerne les mono- ou poly- esters d'acides carboxyliques, ces derniers sont plus particulièrement des esters d'acides gras et/ou d'alcools gras. Ainsi, lesdits esters sont de préférence linéaires ou ramifiés, saturés ou non et comportent avantageusement au moins une chaîne hydrocarbonée comprenant dans la chaîne la plus longue, de 8 à 30 atomes de carbone, plus particulièrement de 8 à 24 atomes de carbone, de préférence de 12 à 24 atomes de carbone, provenant de la partie acide, de la partie alcool.

En outre, ces mono- et poly- esters d'acides carboxyliques peuvent comprendre au moins une chaîne en C₁-C₈, de préférence en C₁-C₆.

De plus, si l'acide carboxylique comprend plusieurs fonctions carboxyliques, celles-ci sont de préférence toutes estérifiées.

Enfin, il est à noter que les alcools sont de préférence des alcools monofonctionnels.

A titre d'exemples, on peut citer les esters des acides oléique, laurique, palmitique, myristique, béhénique, stéarique, linoléique, linolénique, caprique, arachidonique, dérivés de coco ou leurs mélanges comme notamment les mélanges oléo-palmitique, oléo-stéarique, palmito-stéarique, etc.

Conviennnent aussi comme liquide organique inerte, le diester isopropylique de l'acide sébacique (sébaçate de diisopropyle), l'adipate de di-octyle et le maléate de di-caprylyle, ou leurs mélanges.

On peut de plus utiliser un polyester d'un acide polycarboxylique comprenant un radical avec moins de 6 atomes de carbone, saturé ou non, linéaire ou ramifié, et d'un alcool comprenant un radical avec moins de 6 atomes de carbone, saturé ou non, linéaire ou ramifié. A titre d'exemple, on peut citer le citrate de triéthyle.

De préférence, les esters sont choisis parmi ceux obtenus à partir d'acides en C₁₂-C₂₄, plus particulièrement comprenant un groupement carboxylique, et d'un monoalcool saturé, linéaire ou ramifié, en C₃-C₆, ou encore d'un monoalcool saturé, linéaire ou ramifié, en C₁₂-C₂₄.

Selon une variante particulièrement avantageuse de l'invention, la composition de décoloration comprend, à titre de liquide inerte, le stéarate d'octyle dodécyle, le palmitate d'isopropyle, et de préférence le myristate d'isopropyle, seuls ou en mélanges.

Pour ce qui a trait aux mono- ou poly- esters de sucres d'acides en C₈-C₃₀, de préférence en C₁₂-C₂₄, il est précisé que le terme sucre désigne des composés qui possèdent plusieurs fonctions hydroxyle, avec ou sans fonction aldéhyde ou cétone, et qui comportent au moins 4 atomes de carbone. Ces sucres peuvent être des monosaccharides, des oligosaccharides ou des polysaccharides. Comme sucres convenables, on peut citer par exemple le sucrose (ou saccharose), le glucose, le galactose, le ribose, le fucose, le maltose, le fructose, le mannose, l'arabinose, le xylose, le lactose, et leurs dérivés notamment alkylés, tels que les dérivés méthylés comme le méthylglucose.

Quant aux acides en en C₈-C₃₀, saturés ou non, linéaires ou ramifiés, comprenant une ou deux fonctions carboxyliques, on pourra se référer aux listes données auparavant.

Lesdits esters de sucres peuvent être choisis parmi les mono-, di-, tri- et tétra-esters, les polyesters et leurs mélanges.

Plus particulièrement, on préfère utiliser les mono- et di- esters et notamment les mono- ou di- oléates, stéarates, béhénates, oléopalmitates, linoléates, linolénates, oléostéarates, de saccharose, de glucose ou de méthylglucose.

On peut citer, à titre d'exemples, le produit vendu sous la dénomination Glucate DO par la société Amerchol, qui est un dioléate de méthylglucose, les produits vendus sous les dénominations F160, F140, F110, F90, F70, SL40 par la société Crodesta, désignant respectivement les palmito-stéarates de sucrose formés de 73% de monoester et 27% de di- et tri-ester, de 61% de monoester et 39% de di-, tri-, et tétra-ester, de 52% de monoester et 48% de di-, tri-, et tétra-ester, de 45% de monoester et 55% de di-, tri-, et tétra-ester, de 39% de monoester et 61% de di-, tri-, et tétra-ester, et le mono-laurate de sucrose ; les produits vendus sous la dénomination Ryoto Sugar Esters par exemple référencés B370 et correspondant au béhénate de saccharose formé de 20% de monoester et 80% de di-triester-polyester ; le mono-di-palmito-stéarate de sucrose commercialisé par la société Goldschmidt sous la dénomination Tegosoft PSE.

En ce qui concerne les esters et éthers cycliques, on peut citer par exemple la γ-butyrolactone, le diméthyl isosorbide (dénomination CTFA), ou le diisopropyl isosorbide (dénomination CTFA).

Concernant les huiles silicones, on utilise en tant que liquide inerte des composés qui sont plus particulièrement liquides et non volatiles, de préférence de viscosité inférieure ou égale à 10 000 mPa.s à 25°C, la viscosité des silicones étant mesurée selon la norme ASTM 445 Appendice C.

Les huiles de silicone sont définies plus en détail dans l'ouvrage de Walter NOLL "Chemistry and Technology of Silicones" (1968) - Academic Press.

Toutefois, parmi les huiles de silicone convenables, on peut citer notamment les huiles de silicones vendues sous les dénominations DC-200 fluid-5 mPa.s, DC-200 fluid-20 mPa.s, DC-200 fluid-350 mPa.s, DC-200 fluid- 1000 mPa.s, DC-200 fluid-10 000 mPa.s par la société Dow Corning.

L'huile de paraffine est un exemple d'huile minérale susceptible d'être utilisée comme liquide inerte de la composition.

En ce qui concerne les huiles végétales, on peut citer tout particulièrement l'huile d'avocat, l'huile d'olive ou la cire liquide de jojoba.

La teneur en liquide inerte organique de la composition selon l'invention est habituellement comprise entre 0,5 et 35 % en poids, par rapport au poids de la composition.

Selon un premier mode de réalisation particulièrement avantageux de l'invention, la composition de décoloration se trouve sous la forme d'une pâte.

Dans le cadre de ce mode de réalisation, la teneur en liquide organique inerte représente de préférence entre 15 et 35 % en poids, par rapport au poids de la composition.

Selon un deuxième mode de réalisation de l'invention, la composition de décoloration se trouve sous la forme d'un solide divisé.

Dans le cadre de cette variante, la teneur en liquide organique inerte représente de préférence comprise entre 0,5 et moins de 15 % en poids, par rapport au poids de la composition, de préférence compris entre 0,5 et 10 % en poids.

Par ailleurs, la composition de décoloration selon l'invention est anhydre. Plus précisément, la teneur en eau dans la composition est inférieure à 1% en poids, et de préférence inférieure à 0,5% en poids par rapport au poids total de la composition.

De plus, la composition comprend au moins un persulfate.

Plus particulièrement, le ou les persulfates sont choisis parmi les persulfates de métaux alcalino-terreux ou alcalins, comme le magnésium, et de préférence le sodium, le potassium.

Habituellement, la teneur en persulfate dans la composition sous forme de pâte est comprise entre 10 et 70 % en poids par rapport au poids de la composition, de préférence entre 20 et 60 % en poids par rapport au poids de la composition.

La composition selon l'invention peut éventuellement comprendre au moins un agent alcalin. Ce dernier est habituellement choisi parmi l'urée ; les sels d'ammonium, comme les chlorures, les sulfates, les phosphates, les nitrates ; les silicates, les phosphates, les carbonates de métaux alcalins (tels que le sodium, le potassium) ou alcalino-terreux (comme notamment le magnésium) seuls ou combinés.

Habituellement, lorsqu'il est présent, la teneur en agent alcalin dans la composition selon l'invention, est comprise entre 0,01 et 40 % en poids par rapport au poids de la composition, de préférence entre 0,1 et 30 % en poids par rapport au poids de la composition.

De préférence, la composition de décoloration comprend ce type de composé.

La composition selon l'invention, sous forme de pâte, peut de plus comprendre les additifs usuels tels que les polymères amphiphiles comportant au moins une chaîne hydrophobe.

Plus spécialement ces polymères amphiphiles, s'ils sont présents sont de type non ioniques, anioniques, cationiques ou amphotères. De préférence ils sont de nature non ionique, anionique ou cationique.

Lesdits polymères amphiphiles comprennent, plus particulièrement, en tant que chaîne hydrophobe, une chaîne hydrocarbonée, saturée ou non, aromatique ou non, linéaire ou ramifiée, en C₆-C₃₀, comprenant éventuellement un ou plusieurs motifs oxyalkylénés (oxyéthylénés et/ou oxypropylénés).

Parmi les polymères amphiphiles cationique comportant une chaîne hydrophobe on peut trouver des polyuréthannes cationiques ou des copolymères cationiques comprenant des motifs vinyllactame et en particulier vinylpyrrolidone.

Encore plus préférentiellement les polymères amphiphiles comportant une chaîne hydrophobe sont de nature non ionique ou anionique.

A titre d'exemples de polymères amphiphiles non ioniques à chaîne hydrophobe, on peut citer entre autres les celluloses comprenant une chaîne hydrophobe (Natrosol Plus Grade 330 CS® de la société Aqualon ; Bermocoll EHM 100® de la société Berol Nobel ; Amercell Polymer HM-1500® de la société Amerchol) ; les hydroxypropylguars modifiés par un ou plusieurs groupements hydrophobes (Jaguar XC-95/3®, RE210-18, RE205-1, de la société Rhodia Chimie ; Esaflor HM 22® de la société Lamberti) ; les copolymères de vinylpyrrolidone et de monomères à chaîne hydrophobe (certains produits des gammes Antaron®, Ganex® de la société I.S.P) ; les copolymères de (méth)acrylates d'alkyles en C₁-C₆ et de monomères amphiphiles comportant une chaîne hydrophobe ; les copolymères de (méth)acrylates hydrophiles et de monomères comportant au moins une chaîne hydrophobe (copolymère méthacrylate de polyéthylèneglycol / méthacrylate de lauryle) ; les polymères à squelette aminoplaste éther possédant au moins une chaîne grasse (Pure Thix® de la société Süd-Chemie) ; les polyéthers polyuréthannes, linéaires (structure à blocs), greffés ou en étoile, comportant dans leur chaîne, au moins une séquence hydrophile et au moins une séquence hydrophobe (tels que décrits dans l'article de G. Fonnum, J. Bakke et Fk. Hansen - Colloid Polym. Sci 271, 380.389 (1993) ; Nuvis FX 1100® - désignation INCI "Steareth-100/PEG-136/H.M.D.I. Copolymer" de la société Servo Delden ; Rhéolate® 205, 208, 204 ou 212 de la société Rheox ; Elfacos® T210, T212 de la société Akzo).

A titre d'exemples de polymères amphiphiles anioniques comportant au moins une chaîne hydrophobe utilisables dans le cadre de la présente invention, on peut mentionner les polymères, réticulés ou non, comprenant au moins un motif hydrophile dérivé d'un ou de plusieurs monomères à insaturation éthylénique portant une fonction acide carboxylique, libre ou partiellement ou totalement neutralisée, et au moins un motif hydrophobe dérivé d'un ou de plusieurs monomères à insaturation éthylénique portant une chaîne latérale hydrophobe, et éventuellement au moins un motif de réticulation dérivé d'un ou plusieurs monomères polyinsaturés.

Ces polymères sont connus et notamment décrits dans US 3915921 et US 4509949 pour les copolymères d'acide (méth)acrylique et de (méth)acrylates d'alkyles en C₁₀-C₃₀, ou dans EP 216479 pour les copolymères d'acide (méth)acrylique et d'éthers allyliques d'alcools gras).

En outre, les produits Carbopol ETD-2020® et 1382®, Pemulen TR1® et TR2® de la société Goodrich ; le copolymère acide méthacrylique/acrylate d'éthyle/méthacrylate de stéaryle oxyéthyléné (55/35/10) ; le copolymère acide (méth)acrylique/acrylate d'éthyle/méthacrylate de béhényle oxyéthyléné 25 OE ; le copolymère réticulé acide méthacrylique/acrylate d'éthyle/stéareth-10 allyl éther, sont des polymères qui conviennent à la mise en oeuvre de l'invention.

Si ces polymères amphiphiles sont présents, leur teneur représente de 0,01 à 30 % en poids par rapport au poids de la composition.

La composition de décoloration selon invention peut en outre comprendre au moins un polymère épaississant dépourvu de chaîne hydrophobe.

En ce qui concerne les polymères ne comprenant pas de chaîne hydrophobe au sens détaillé précédemment, ceux-ci peuvent être d'origine naturelle, ou être des polymères synthétiques, et sont avantageusement choisis parmi ceux utilisés classiquement dans le domaine cosmétique.

Comme exemples de polymères synthétiques, on peut citer, la polyvinylpyrrolidone, l'acide polyacrylique, le polyacrylamide, l'acide poly-2-acrylamidopropanesulfonique non réticulé (Simugel® EG de la société Seppic), l'acide poly-2-acrylamido-2-méthylpropane sulfonique réticulé, libre ou partiellement neutralisé par l'ammoniaque (Hostacerin® AMPS de la société Clariant), des mélanges d'acide poly-2-acrylamido-2-méthylpropane sulfonique non réticulé avec des éthers d'hydroxyalkylcellulose ou avec des poly(oxyde d'éthylène) tel qu'ils sont décrits dans le brevet US 4540510 ; des mélanges d'acide poly(méth)acrylamidoalkyl(C₁-C₄)-sulfonique, de préférence réticulé, avec un copolymère réticulé de l'anhydride maléique et d'un alkyl(C₁-C₅)vinytéther (Stabileze QM de la société ISF).

Les polymères épaississants d'origine naturelle utilisables selon la présente invention, sont de préférence des polymères comportant au moins un motif sucre, comme les gommes de guar non ioniques, modifiées ou non par des groupements hydroxyalkyle en C₁-C₆; les gommes de biopolysaccharides d'origine microbienne telles que les gommes de Scléroglucane ou de Xanthane ; les gommes issues d'exudats végétaux telles que les gommes Arabique, Ghatti, Karaya, Tragacanthe, Carraghénanne, Agar et Caroube ; les pectines ; les alginates ; les amidons ; les hydroxyalkyl(C₁-C₆)celluloses et carboxyalkyl(C₁-C₆)celluloses, ou encore leurs mélanges.

Là encore, "motif sucre" désigne une portion monosaccharidique [soit monosaccharide ou oside ou sucre simple], oligosaccharidique [soit chaînes courtes formées de l'enchaînement d'unités monosaccharidiques, éventuellement différentes] ou polysaccharidique [soit longues chaînes constituées d'unités monosaccharidiques, éventuellement différentes, i.e. polyholosides ou polyosides]. Les unités saccharidiques peuvent être en outre substituées par des radicaux alkyle, ou hydroxyalkyle, ou alcoxy, ou acyloxy, ou carboxyle, les radicaux alkyle comportant de 1 à 4 atomes de carbone.

Les polymères épaississants dépourvus de chaîne hydrophobe, s'ils sont présents, représentent de 0,01 à 30 % en poids de la composition de décoloration anhydre.

La composition selon l'invention peut éventuellement comprendre au moins un tensioactif, de préférence non ionique, anionique, amphotère ou zwittérionique.

Parmi les tensioactifs non ioniques, on peut citer les alcools, les alpha-diols, les alkylphénols polyéthoxylés et/ou polypropoxylés, ayant une chaîne hydrocarbonée comprenant par exemple de 8 à 30 atomes de carbone, de préférence de 8 à 18 atomes de carbone, le nombre de groupements oxyde d'éthylène et/ou oxyde de propylène pouvant aller notamment de 2 à 50.

On peut également citer les copolymères d'oxyde d'éthylène et de propylène, les condensats d'oxyde d'éthylène et/ou de propylène sur des alcools gras ; les amides gras polyéthoxylés ayant de préférence de 2 à 30 moles d'oxyde d'éthylène ; les esters d'acides gras de sorbitan oxyéthylénés ayant de 2 à 30 moles d'oxyde d'éthylène ; les esters d'acides gras de sucrose, les esters d'acides gras et de polyéthylèneglycol, les alkylpolyglycosides, les dérivés de N-alkyl glucamine, etc.

Parmi les tensioactifs anioniques, on peut citer entre autres les sels (en particulier les sels alcalins, notamment de sodium, de magnésium, les sels d'ammonium, les sels d'amines, les sels d'aminoalcools) d'alkylsulfates, alkyléthersulfates, alkylamidoéthersulfates, alkylarylpolyéthersulfates, monoglycérides sulfates ; alkylsulfonates, alkylphosphates, alkylamidesulfonates, alkylarylsulfonates, α-oléfine-sulfonates, paraffine-sulfonates ; alkyl(C₆-C₂₄) sulfosuccinates, alkyl(C₆-C₂₄) éthersulfosuccinates, alkyl(C₆-C₂₄) amidesulfosuccinates ; alkyl(C₆-C₂₄) sulfoacétates ; acyl(C₆-C₂₄) sarcosinates et glutamates ; esters d'alkyl(C₆-C₂₄)polyglycosides carboxyliques ; alkylsulfosuccinamates ; acyliséthionates et N-acyltaurates ; le radical alkyle ou acyle de tous ces différents composés comportant de préférence de 12 à 20 atomes de carbone, et le radical aryle désignant de préférence un groupement phényle ou benzyle.

Conviennent aussi les sels d'acides gras tels que les sels des acides oléique, ricinoléique, palmitique, stéarique, les acides d'huile de coprah hydrogénée ou non ; les acides d'alkyl D-galactoside uroniques et leurs sels, les acides alkyl (C₆-C₂₄) éther carboxyliques polyoxyalkylénés, les acides alkyl(C₆-C₂₄)aryl éther carboxyliques polyoxyalkylénés, les acides alkyl(C₆-C₂₄) amido éther carboxyliques polyoxyalkylénés et leurs sels, en particulier ceux comportant de 2 à 50 groupements oxyde d'alkylène en particulier d'éthylène, et leurs mélanges.

En ce qui concerne les tensioactifs amphotères ou zwittérioniques, conviennent notamment les dérivés d'amines secondaires ou tertiaires aliphatiques, dans lesquels le radical aliphatique est une chaîne linéaire ou ramifiée comportant 8 à 18 atomes de carbone et contenant au moins un groupe anionique hydrosolubilisant (par exemple carboxylate, sulfonate, sulfate, phosphate ou phosphonate) ; on peut citer encore les alkyl (C₈-C₂₀) bétaïnes, les sulfobétaïnes, les alkyl (C₈-C₂₀) amidoalkyl (C₁-C₆) betaïnes ou les alkyl (C₈-C₂₀) amidoalkyl (C₁-C₆) sulfobétaïnes.

Parmi les dérivés d'amines, on peut citer amphocarboxyglycinates et amphocarboxypropionates, comme par exemple les disodium cocoamphodiacetate, disodium lauroamphodiacetate, disodium caprylamphodiacetate, disodium cocoamphodipropionate, disodium lauroamphodipropionate, etc. (produits de la gamme Miranol®, notamment Miranol^{®} C2M concentré commercialisés la société Rhodia Chimie).

Au cas où un ou plusieurs tensioactifs seraient présents dans la composition sous forme de pâte, leur teneur est de préférence telle que la teneur en tensioactif dans la composition prête à l'emploi, qui sera décrite plus loin, représente de 0,05 à 30 % en poids par rapport au poids de la composition prête à l'emploi, et de préférence 0,1 à 20 % en poids par rapport au poids de cette composition.

La composition sous forme de pâte selon l'invention peut de plus comprendre un ou plusieurs agents gélifiants choisis par exemple parmi les silices pyrogénées à caractère hydrophile (notamment Aerosil^{®} 90, 130, 150, 200, 300 et 380, société Degussa Hüls), parmi les silices pyrogénées à caractère hydrophobe (notamment Aerosil^{®} R202, R805, R812, R972 et R974, société Degussa Hüls). Parmi les agents gélifiants conviennent aussi les copolymères di-, tri-, multi- ou radial blocs composés de segments de type styrène monomère et de segments de type monomère ou comonomère thermoplastique, comme, entre autres, ceux décrits dans le brevet US-5 221 534. Parmi ces copolymères blocs, on préfère utiliser notamment ceux pour lesquels le monomère ou comonomère thermoplastique désigne éthylène/alkylène en C₃-C₄, et plus particulièrement encore un copolymère hydrogéné à blocs styrène et à blocs éthylène/alkylène en C₃-C₄ (Versagel® M200 et Geahlene® 200, Versagel® M750 et Geahlene® 750, société Penreco ; Transgel®, Syngel®, société Aiglon). On peut également utiliser les polymères tri-blocs styrène /éthylene-butylène / styrène (Kraton® G-1650, G-1652 et G-1657, société Shell Chimie).

S'ils sont présents, la teneur en agents gélifiants représente de préférence une teneur comprise entre 0,01 et 10% en poids par rapport au poids de la composition sous forme de pâte, de préférence entre 0,01 et 5% en poids par rapport à la même référence.

La composition sous forme de pâte conforme à l'invention peut également comprendre divers adjuvants utilisés classiquement, tels que des polymères substantifs cationiques ou amphotères, des polymères anioniques, cationiques, non ioniques, amphotères, zwittérioniques ; des agents épaississants minéraux ; des agents antioxydants ; des agents de pénétration ; des parfums ; des tampons ; des agents dispersants ; des agents peptisants ; des agents de conditionnement tels que par exemple des cations ; des silicones volatiles ou non volatiles, modifiées ou non modifiées ; des agents filmogènes ; des céramides, des vitamines ou provitamines ; des agents conservateurs ; des agents stabilisants ; des agents opacifiants ou matifiants comme le dioxyde de titane ; des charges minérales, comme les argiles ; des polymères liants tels que la vinylpyrrolidone ; des lubrifiants comme les stéarates de polyols ou les stéarates de métaux alcalins ou alcalino-terreux ; des agents de contrôle du dégagement d'oxygène tels que le carbonate ou l'oxyde de magnésium, etc.

Les adjuvants cités ci-dessus sont en général présents en quantité comprise pour chacun d'eux entre 0,01 et 20% en poids par rapport au poids de la composition.

La composition de décoloration qui vient d'être détaillée peut être préparée de manière classique, sous agitation mécanique, de l'ensemble des éléments constitutifs.

Plus spécialement, dans le cas où ladite composition se trouve sous la forme d'une pâte, celle-ci peut être préparée en dispersant, sous agitation mécanique, l'ensemble des composés pulvérulents dans le liquide inerte, dans lequel on a préalablement dispersé ou mélangé les autres composés liquides de la composition de décoloration.

On peut également préparer la pâte par extrusion, en introduisant les phases liquides et solides de la composition dans l'extrudeur, puis en les mélangeant à une température inférieure à 25°C à l'aide d'un système bi-vis co-rotatives composé d'éléments de transport et de malaxage.

Un autre objet de la composition est constitué par une composition prête à l'emploi susceptible d'être obtenue par mélange de la composition qui vient d'être décrite, avec une composition comprenant au moins un agent oxydant.

De préférence, l'agent oxydant est le peroxyde d'hydrogène.

Conformément à un mode de réalisation particulier de l'invention, la teneur en peroxyde d'hydrogène dans la composition prête à l'emploi, représente de 1 à 12 % en titre en peroxyde d'hydrogène, plus particulièrement de 2 à 9% en titre en peroxyde d'hydrogène, de préférence de 2 à 6 % en titre en peroxyde d'hydrogène.

Il est à noter que la teneur en composé choisi parmi l'acide éthylènediamine-N,N'-disuccinique, sel ou mélange, dans la composition prête à l'emploi, représente plus particulièrement de 0,05 à 10 % en poids par rapport au poids de cette composition, avantageusement de 0,5 à 5 % en poids, et de préférence de 0,1 à 2,5 % en poids par rapport au poids de la composition prête à l'emploi.

Par ailleurs, selon une variante de l'invention, la teneur en persulfate dans la composition prête à l'emploi est plus particulièrement comprise entre 5 et 35 % en poids par rapport au poids de la composition prête à l'emploi, de préférence entre 10 et 30 % en poids par rapport à la même référence.

Quant à la teneur en agent alcalin dans la composition prête à l'emploi, cette dernière représente avantageusement de 0,005 et 20 % en poids par rapport au poids de ladite composition prête à l'emploi, de préférence entre 0,05 et 15 % en poids par rapport au poids de cette composition.

Comme indiqué auparavant, la composition prête à l'emploi est obtenue par mélange avant l'emploi (c'est-à-dire avant l'application de cette composition prête à l'emploi sur les fibres à décolorer) d'une composition de décoloration telle que décrite auparavant, avec une composition comprenant au moins un agent oxydant.

Plus particulièrement, la teneur en peroxyde d'hydrogène dans la composition oxydante est avantageusement comprise entre 1 et 12 % en titre en peroxyde d'hydrogène, plus particulièrement comprise entre 2 et 12 % en titre en peroxyde d'hydrogène, et de préférence entre 2,7 et 12 % en titre en peroxyde d'hydrogène.

Il est à noter que la composition comprenant agent oxydant peut se trouver sous la forme d'une solution, d'une émulsion ou d'un gel.

Selon un mode de réalisation préféré de l'invention, la composition comprenant l'agent oxydant se trouve sous la forme d'une émulsion huile dans eau.

De préférence, l'émulsion huile dans eau de peroxyde d'hydrogène, comprend au moins un tensioactif non ionique et/ou anionique.

Les tensioactifs dont une liste a été donnée précédemment dans le cadre de la description de la composition sous forme de pâte, est valable dans le cadre de l'émulsion huile dans eau et l'on pourra s'y référer.

Cependant, selon une variante préférée de l'invention, les tensioactifs présents dans l'émulsion huile dans eau comprenant l'agent oxydant, sont choisis parmi les alkylsulfates, les alkyléthersulfates de métal alcalin comme le sodium, le potassium, alcalino-terreux comme le magnésium, d'ammonium, d'amines, d'aminoalcools.

Il peut de même être avantageux de mettre en oeuvre au moins un tensioactif non ionique choisi par exemple parmi, seuls ou en mélanges, les alcools gras (notamment en C₆-C₂₄) polyéthoxylés et/ou polypropoxylés, le nombre de groupements oxyde d'éthylène et/ou oxyde de propylène étant habituellement compris entre 1 et 50 ; les alcools gras (notamment en C₆-C₂₄) mono- ou poly-glycérolés comportant en moyenne 1 à 30 groupements glycérol et les amides gras (notamment en C₆-C₂₄) polyglycérolés comportant en moyenne 1 à 5 groupements glycérol et en particulier 1,5 à 4.

Il est à noter que la teneur en tensioactifs présents dans la composition oxydante est en général comprise entre 0,05 et 30 % en poids, de préférence entre 0,1 et 20 % en poids par rapport à ladite composition oxydante.

De préférence, et comme indiqué auparavant, la composition prête à l'emploi présente une teneur totale en tensioactifs comprise entre 0,05 et 30 % en poids par rapport au poids de la dite composition prête à l'emploi, et de préférence entre 0,1 et 20 % en poids par rapport à la même référence.

Conformément à un mode avantageux de l'invention, la composition comprenant l'agent oxydant peut comprendre au moins un copolymère, réticulé ou non, présentant au moins un motif provenant d'un monomère à insaturation éthylénique à groupement sulfonique, sous forme libre ou partiellement ou totalement neutralisée et comprenant au moins une partie hydrophobe. De préférence, les monomères sont choisis parmi les acides (méth)acrylamido(C₁-C₂₂) alkylsulfoniques tels que par exemple l'acide acrylamido-méthane-sulfonique, l'acide acrylamido-éthane-sulfonique, l'acide acrylamido-propane-sulfonique, l'acide 2-acrylamido-2-méthylpropane-sulfonique (AMPS), l'acide méthacrylamido-2-méthylpropane-sulfonique, l'acide 2-acrylamido-n-butane-sulfonique, l'acide 2-acrylamido-2,4,4-triméthylpentane-sulfonique, l'acide 2-méthacrylamido-dodécyl-sulfonique, l'acide 2-acrylamido-2,6-diméthyl-3-heptane-sulfonique ainsi que leurs formes partiellement ou totalement neutralisées.

La neutralisation totale ou partielle des fonctions sulfoniques du copolymère peut être faite au moyen d'une base minérale, telle que la soude, la potasse, ammoniaque ; ou d'une base organique telle que la mono-, di- ou tri-éthanolamine, un aminométhyl-propanediol, la N-méthyl-glucamine, les acides aminés basiques comme l'arginine et la lysine, et les mélanges de ces composés.

La partie hydrophobe du copolymère comporte plus particulièrement 8 à 30 atomes de carbone, de préférence 8 à 22 atomes de carbone, et de manière encore plus avantageuse 8 à 18 atomes de carbone, et encore plus particulièrement 12 à 18 atomes de carbone.

Ces copolymères peuvent plus particulièrement être choisis parmi les polymères amphiphiles statistiques d'AMPS modifiés par réaction avec une n-monoalkylamine ou une di-n-alkylamine en C₆-C₂₂, et tels que ceux décrits dans la demande de brevet WO00/31154, dont la description des polymères et de leur synthèse fait partie de la description.

Ces copolymères peuvent également contenir d'autres monomères hydrophiles éthyléniquement insaturés choisis par exemple parmi les acides (méth)acryliques, leurs dérivés alkyl substitués en β ou leurs esters obtenus avec des monoalcools ou des mono- ou poly- alkylèneglycols, les (méth)acrylamides, la vinylpyrrolidone, l'anhydride maléique, l'acide itaconique ou l'acide maléique ou les mélanges de ces composés.

Les polymères préférés sont choisis notamment parmi les copolymères amphiphiles d'AMPS et d'au moins un monomère hydrophobe à insaturation éthylénique (voir par exemple EP750899, US 5089578, FR 2 818 543 et dans les publications de Yotaro Morishima suivantes : "Self-assembling amphiphilic polyelectrolytes and their nanostructures - Chinese Journal of Polymer Science Vol. 18, N°40, (2000), 323-336" ; "Miscelle formation of random copolymers of sodium 2-(acrylamido)-2-methylpropanesulfonate and a non-ionic surfactant macromonomer in water as studied by fluorescence and dynamic light scattering - Macromolecules 2000, Vol. 33, N° 10 - 3694-3704" ; "Solution properties of miscelle networks formed by non-ionic moieties covalently bound to an polyelectrolyte : salt effects on rheological behavior - Langmuir, 2000, Vol.16, N°12, 5324-5332" ; "Stimuli responsive amphiphilic copolymers of sodium 2-(acrylamido)-2-methylpropanesulfonate and associative macromonomers - Polym. Preprint, Div. Polym. Chem. 1999, 40(2), 220-221".

Parmi ces copolymères, on peut citer les copolymères réticulés ou non, neutralisés ou non, comportant de 15 à 60% en poids de motifs AMPS et de 40 à 85% en poids de motifs (C₈-C₁₆)alkyl(méth)acrylamide ou de motifs (C₈-C₁₆)alkyl(méth)acrylate par rapport au copolymère, tels que ceux décrits dans la demande EP-A750 899 ; les terpolymères comportant de 10 à 90% en mole de motifs acrylamide, de 0,1 à 10% en mole de motifs AMPS et de 5 à 80% en mole de motifs n-(C₆-C₁₈)alkylacrylamide, tels que ceux décrits dans le brevet US- 5089578 ; les copolymères réticulés ou non d'AMPS partiellement ou totalement neutralisés et de méthacrylate de dodécyle ou de n-dodécylméthacrylamide, tels que ceux décrits dans les articles de Morishima cités ci-dessus.

Selon un mode de réalisation particulier de l'invention, et s'il est présent, la teneur en copolymère représente 0,005 à 15 % en poids de la composition prête à l'emploi, plus particulièrement de 0,05 à 7,5 % en poids de la composition prête à l'emploi, de préférence de 0,1 à 5 % en poids de la composition prête à l'emploi.

La phase huile de l'émulsion de la composition comprenant l'agent oxydant comprend de préférence au moins un alcool gras, saturé ou insaturé, linéaire ou ramifié, comprenant de 12 à 30 atomes de carbone, de préférence 12 à 22 atomes de carbone. On peut citer plus particulièrement parmi eux, les alcools laurique, cétylique, stéarylique, oléïque, béhénique, linoléïque, undécylénique, palmitoléïque, linolénique, arachidonique, érucique, et leurs mélanges.

Dans de telles émulsions, la concentration en alcools gras peut varier d'environ 0,1 à 30% en poids et plus préférentiellement d'environ 0,5 à 15% en poids par rapport au poids total de l'émulsion.

La composition comprenant l'agent oxydant peut de même comprendre les additifs usuels dans le domaine, comme par exemple des agents stabilisants l'eau oxygénée tels les sels de stannate et de pyrophosphate de métaux alcalins (comme le sodium, le potassium par exemple), ou le salicylate de sodium ; des colorants ; des parfums ; des agents anti-mousse ; des polymères substantifs cationiques ou amphotères.

Plus particulièrement, le pH de la composition comprenant l'agent oxydant, est compris entre 1 et 6, et préférentiellement entre 2 et 4.

Le pH acide garantit la stabilité de l'agent oxydant quand celui-ci est le peroxyde d'hydrogène. Le pH peut être réglé à l'aide d'agents acidifiants tels que par exemple l'acide chlorhydrique, l'acide acétique, l'acide phosphorique, l'acide lactique, l'acide citrique, l'acide salicylique ou l'acide borique. En outre, le pH peut être ajusté classiquement et si nécessaire par ajout d'agents alcalinisants, tels que par exemple, l'ammoniaque, la monoéthanolamine, la diéthanolamine, la triéthanolamine, l'isopropanolamine, le 1,3-diamino-propane, un (bi)carbonate alcalin ou d'ammonium, un carbonate organique tel que le carbonate de guanidine, ou bien encore un hydroxyde alcalin, tous ces composés pouvant bien entendu être pris seuls ou en mélange.

La composition comprenant l'agent oxydant est préparée de manière classique. Plus particulièrement, au cas où cette composition se trouve sous la forme d'une émulsion, elle peut être préparée en mélangeant à température ambiante le peroxyde d'hydrogène et les autres ingrédients de la phase aqueuse de l'émulsion huile dans eau puis de réaliser l'émulsion par ajout de la phase huile de l'émulsion, à une température supérieure à la température ambiante.

Le procédé de préparation de la composition de décoloration prête à l'emploi selon l'invention consiste donc à mélanger la composition de décoloration et la composition comprenant l'agent oxydant. Ce mélange doit se faire peu avant l'application de la composition prête à l'emploi sur les fibres à décolorer. Le mot "peu" s'entend généralement comme une période de temps comprise entre quelques minutes à quelques dizaines de minutes.

Habituellement, on mélange la composition de décoloration avec environ 0,5 à 10 équivalents en poids de la composition comprenant l'agent oxydant.

Notons que le pH de la composition prête à l'emploi selon l'invention est, avantageusement compris entre 4 et 12, plus particulièrement entre 7 et 11,5, et de préférence entre 8 et 11.

La présente invention a de même pour objet un procédé de décoloration de fibres kératiniques humaines, en particulier des cheveux, consistant à appliquer sur les fibres kératiniques humaines à décolorer, sèches ou humides, la composition prête à l'emploi ; à la laisser agir pendant un temps de pause suffisant pour obtenir la décoloration recherchée ; puis à éliminer la composition par un rinçage à l'eau.

De manière préférée, le rinçage à l'eau est suivi d'un lavage avec un shampooing et d'un rinçage à l'eau, puis éventuellement d'un séchage.

Habituellement, le temps de pause de la composition varie entre 1 et 60 minutes, plus préférentiellement entre 10 et 50 minutes.

La température à laquelle la composition est laissée agir est en général comprise entre la température ambiante (15 à 25°C) et 220°C, plus particulièrement entre la température ambiante et 80°C, de préférence entre 15 et 40 °C.

L'invention a enfin pour objet un dispositif à plusieurs compartiments, ou "kit", pour la mise en oeuvre du procédé de décoloration des fibres kératiniques humaines.

Ce dispositif comporte au moins deux compartiments dont l'un contient une composition sous forme de pâte comprenant au moins au moins un composé choisi parmi l'acide éthylènediamine-N,N'-disuccinique, un sel, ou leur mélange, au moins un liquide inerte organique, au moins un persulfate, au moins un agent alcalin ; et l'autre contient une composition oxydante.

Des exemples non limitatifs de la présente invention vont maintenant être présentés.

### EXEMPLE 1

### Compositions prêtes à l'emploi de décoloration :

| | ***Quantités (en g%)*** | |
|---|---|---|
| **Dénomination Matières Premières** | **Composition A** | **Composition B** |
| Persulfate de potassium | 27,8 | 24,4 |
| Disilicate de sodium | 5,6 | 7,6 |
| Chlorure d'ammonium | 2,4 | 1,6 |
| Urée | / | 1,8 |
| Acide éthylènediamine disuccinique, sel trisodique en solution aqueuse (Octaquest E30® ; Société Octel) | 0,4 | 0,32 |
| Copolymère hexaméthyl di-isocyanate / polyéthylène glycol à terminaison α et ω stéaryl polyoxyéthylène (Nuvis FX 1100® ; Société Servo Delden) | 0,5 | / |
| Copolymère acide acrylique / métacrylate d'alkyl (C₁₀ / C₃₀) réticulé (Carbopol ETD 2020® ; Société Noveon) | / | 0,4 |
| Carboxyméthyl amidon de pomme de terre / sel de sodium faiblement réticulé | / | 0,8 |
| Gomme de guar | 0,8 | 1 |
| Colorant (ultramarine) | 0,2 | / |
| Oxyde de titane | 0,2 | 0,4 |
| Lauryl sulfate de sodium | 0,8 | 0,8 |
| Stéarate de calcium | 0,4 | 0,4 |
| Silice pyrogénée à caractère hydrophile | 1,2 | 0,1 |
| Polydécène hydrogéné (Silkflo 366 NF Polydecene ; Société Amoco Chemical) | 2 | 0,8 |
| Eau oxygénée à 9% | 10,8 | 10,8 |
| Alcool cétylique | 0,5 | 0,5 |
| Lauryl Sulfate de Sodium | 0,2 | 0,2 |
| Alcool oléique polyglycérolé (2 moles) | 0,4 | 0,4 |
| Eau | Qsp 100 g | Qsp 100 g |

On applique la composition A, 45 minutes sous casque sur cheveux naturels foncés, puis on rince abondamment à l'eau.

On obtient une décoloration puissante et homogène.

On applique la composition B, 30 minutes sous casque sur cheveux naturels foncés, puis on rince abondamment à l'eau.

On obtient une décoloration puissante et homogène, avec des cheveux doux et brillants, et faciles à démêler.

### EXEMPLE 2

### Composition oxydante pour un usage en décoloration :

| | ***Quantités (en g%)*** |
|---|---|
| **Dénomination Matières Premières** | **Composition C** |
| Alcool cétylique | 3 |
| Lauryl sulfate de sodium | 0,5 |
| Alcool oléique polyglycérolé (2 moles) | 0,8 |
| Simethicone | 0,045 |
| Acide éthylènediamine disuccinique, sel trisodique en solution aqueuse (Octaquest E30® ; Société Octel) | 0,13 |
| Copolymère AMPS/méthacrylate de cétéaryl éthoxylé (25 OE) 80/20, réticulé par triméthylolpropane triacrylate ; (Aristoflex HMS® ; Société Clariant) | 0,05 |
| Pyrophosphate tétrasodique, 10 H₂O | 0,02 |
| Salicylate de sodium | 0,01 |
| Stannate de sodium, 6 H₂O | 0,04 |
| Eau oxygénée à 50 % | 12 |
| Acide phosphorique à 85 % en solution aqueuse | Qsp pH = 2 |
| Eau | Qsp 100 g |

### Compositions anhydres oxydantes de décoloration sous forme de pâtes :

| | ***Quantités (en g%)*** | |
|---|---|---|
| **Dénomination Matières Premières** | **Composition D** | **Composition E** |
| Persulfate de potassium | 41,8 | 41,6 |
| Disilicate de sodium | 18 | 18 |
| Chlorure d'ammonium | 4,2 | 4,2 |
| Séquestrant | 1 | 1 |
| Copolymère hexaméthyl di-isocyanate / polyéthylène glycol à terminaison α et ω stéaryl polyoxyéthylène (Nuvis FX 1100® ; Société Servo Delden) | 2 | 0,5 |
| Copolymère acide acrylique / métacrylate d'alkyl (C₁₀ / C₃₀) réticulé (Carbopol ETD 2020® ; Société Noveon) | / | 0,5 |
| Carboxyméthyl amidon de pomme de terre / sel de sodium faiblement réticulé | 2 | 1 |
| Gomme de guar | / | 2 |
| Colorant (ultramarine) | 0,5 | 0,5 |
| Oxyde de titane | 1 | 1 |
| Lauryl sulfate de sodium | 3,5 | 3,5 |
| Stearate de calcium | 2 | 2 |
| Silice pyrogénée à caractère hydrophile | 0,5 | 0,5 |
| Palmitate d'isopropyle | 22,5 | / |
| Cire d'abeille | 1 | / |
| Polydécène hydrogéné (Silkflo® 366 NF Polydecene ; Société Amoco Chemical) | / | 23 |
| Silice pyrogénée à caractère hydrophobe | / | 0,7 |

On mélange 40 g de la composition de décoloration D avec 80 g de la composition oxydante à base d'eau oxygénée C.

On applique le mélange décolorant prêt à l'emploi ainsi obtenu pendant un temps de pose de 45 minutes sous casque, sur cheveux naturels foncés, puis on rince abondamment à l'eau.

On obtient une décoloration puissante et homogène, avec des cheveux doux et brillants, et faciles à démêler.

On mélange 40 g de la composition de décoloration E avec 60 g de la composition oxydante à base d'eau oxygénée C.

On applique le mélange décolorant prêt à l'emploi ainsi obtenu pendant un temps de pose de 25 minutes sous casque, sur cheveux naturels foncés, puis on rince abondamment à l'eau.

On obtient une décoloration puissante et homogène, avec des cheveux doux et brillants, et faciles à démêler.

## Revendications

1. Composition de décoloration des fibres kératiniques humaines, **caractérisée en ce qu'**elle comprend :
- au moins au moins un composé choisi parmi l'acide éthylènediamine-N,N'-disuccinique, un de ses sels, ou leur mélange.
- au moins un liquide inerte organique,
- au moins un persulfate.

2. Composition selon la revendication précédente, **caractérisée en ce que** la teneur en composé est comprise entre 0,01 et 20 % en poids par rapport au poids de la composition, plus particulièrement entre 0,1 et 10 % en poids par rapport au poids de la composition, de préférence entre 0,2 et 5 % en poids par rapport au poids de la composition.

3. Composition selon l'une des revendications, précédentes, **caractérisée en ce que** le liquide inerte organique comprend au moins un composé choisi parmi les polydécènes, les mono- et poly- esters d'acides carboxyliques, les mono- ou poly- esters de sucres d'acides en C₈-C₃₀, les éthers cycliques, les esters cycliques, les huiles silicones, les huiles minérales, les huiles végétales, ou leurs mélanges.

4. Composition selon l'une des revendications précédentes, **caractérisée en que** le liquide inerte est choisi parmi les esters d'acides en C₈-C₃₀ et d'un monoalcool saturé, linéaire ou ramifié, en C₃-C₆.

5. Composition selon l'une quelconque des revendications précédentes, **caractérisée en ce** teneur en liquide inerte représente de 0,5 à 35 % en poids par rapport au poids de la composition.

6. Composition selon l'une quelconque des revendications précédentes, **caractérisée en ce que** la composition de décoloration se trouve sous la forme d'une pâte.

7. Composition selon la revendication précédente, **caractérisée en ce** teneur en liquide inerte représente de 15 à 35 % en poids par rapport au poids de la composition.

8. Composition selon l'une quelconque des revendications précédentes, **caractérisée en ce que** la composition de décoloration se trouve sous la forme d'un solide divisé.

9. Composition selon la revendication précédente, **caractérisée en ce** teneur en liquide inerte représente de 0,5 à moins de 15 % en poids par rapport au poids de la composition.

10. Composition selon l'une quelconque des revendications précédentes, **caractérisée en que** le persulfate est choisi parmi les persulfates de sodium et de potassium, seuls ou en mélange.

11. Composition selon l'une quelconque des revendications précédentes, **caractérisée en ce que** la teneur en persulfate est comprise entre 10 et 70 % en poids par rapport au poids de la composition, de préférence entre 20 et 60 % en poids par rapport au poids de la composition.

12. Composition selon l'une quelconque des revendications précédentes, **caractérisée en ce qu'**elle comprend au moins un agent alcalin.

13. Composition selon la revendication précédente, **caractérisée en ce que** l'agent alcalin est choisi parmi l'urée ; les sels d'ammonium ; les silicates, les phosphates, les carbonates de métaux alcalins ou alcalino-terreux, seuls ou en mélange.

14. Composition selon l'une des revendications 12 ou 13, **caractérisée en ce que** la teneur en agent alcalin est comprise entre 0,01 et 40 % en poids par rapport au poids de la composition, de préférence entre 0,1 et 30 % en poids par rapport au poids de la composition.

15. Composition selon l'une des revendications précédentes, **caractérisée en ce que** la composition contient au moins un agent polymère épaississant comprenant ou non une chaîne hydrophobe.

16. Composition selon la revendication 15, **caractérisée en ce que** la teneur en agent épaississant représente 0,01 à 30 % en poids par rapport au poids de la composition.

17. Composition selon l'une des revendications précédentes, **caractérisée en ce que** la composition comprend moins de 1 % en poids d'eau, et de préférence moins de 0,5 % en poids d'eau.

18. Composition de décoloration prête à l'emploi susceptible d'être obtenue par mélange de la composition de décoloration selon l'une quelconque des revendications précédentes, avec une composition comprenant au moins un agent oxydant.

19. Composition prête à l'emploi selon la revendication précédente **caractérisée en ce que** l'agent oxydant est le peroxyde d'hydrogène.

20. Composition prête à l'emploi selon la revendication précédente, **caractérisée en ce que** la teneur en peroxyde d'hydrogène dans la composition prête à l'emploi, représente de 1 à 12 % en titre en peroxyde d'hydrogène, plus particulièrement de 2 à 9% en titre en peroxyde d'hydrogène, de préférence de 2 à 6 % en titre en peroxyde d'hydrogène.

21. Composition prête à l'emploi selon quelconque l'une des revendications 18 à 20, **caractérisée en ce que** la teneur en composé choisi parmi l'acide éthylènediamine-N,N'-disuccinique, sel ou mélange, représente de 0,05 à 10 % en poids par rapport au poids de cette composition, avantageusement de 0,5 à 5 % en poids, et de préférence de 0,1 à 2,5 % en poids par rapport au poids de la composition prête à l'emploi.

22. Composition prête à l'emploi selon l'une quelconque des revendications 19 à 21, **caractérisée en ce que** la teneur en persulfate est comprise entre 5 et 35 % en poids par rapport au poids de la composition prête à l'emploi, de préférence entre 10 et 30 % en poids par rapport au poids de la composition prête à l'emploi.

23. Composition prête à l'emploi selon l'une quelconque des revendications 19 à 22, **caractérisée en ce que** la teneur en agent alcalin est comprise entre 0,005 et 20 % en poids par rapport au poids de la composition prête à l'emploi, de préférence entre 0,05 et 15 % en poids par rapport au poids de la composition prête à l'emploi.

24. Composition prête à l'emploi selon l'une quelconque des revendications 19 à 23, **caractérisée en ce qu'**elle comprend au moins un tensioactif non ionique, anionique, amphotère, zwitterionique ou cationique, ou leurs mélanges.

25. Composition prête à l'emploi selon l'une des revendications 19 ou 24, **caractérisée en ce que** la teneur totale en tensioactif représente 0,05 et 30 % en poids par rapport au poids de la composition prête à l'emploi, de préférence entre 0,1 et 20 % en poids par rapport au poids de la composition prête à l'emploi.

26. Composition prête à l'emploi selon l'une des revendications 19 à 25, **caractérisée en ce que** le pH de la composition prête à l'emploi est compris entre 4 et 12, plus particulièrement entre 7 et 11,5, de préférence entre 8 et 11.

27. Procédé de décoloration de fibres kératiniques humaines, en particulier des cheveux, consistant à appliquer la composition prête à l'emploi selon l'une quelconque des revendications 18 à 26, sur les fibres kératiniques humaines à décolorer, sèches ou humides ; à la laisser agir pendant un temps de pause suffisant pour obtenir la décoloration recherchée ; à éliminer la composition par un rinçage à l'eau.

28. Procédé selon la revendication précédente, **caractérisée en ce qu'**après le rinçage à l'eau, on effectue un lavage des fibres traitées avec un shampooing, puis un rinçage à l'eau et éventuellement un séchage.

29. Procédé selon l'une des revendications 27 ou 28, **caractérisé en ce que** l'on prépare la composition prête à l'emploi, avant son application sur les fibres, en mélangeant la composition de décoloration selon l'une quelconque des revendications 1 à 17 avec une composition comprenant au moins un agent oxydant.

30. Dispositif à plusieurs compartiments pour la mise en oeuvre du procédé selon l'une des revendications 27 à 29, **caractérisé par le fait qu'**il comporte au moins deux compartiments dont l'un contient une composition de décoloration comprenant au moins un composé choisi parmi l'acide éthylènediamine-N,N'-disuccinique, un de ses sels, ou leur mélange ; au moins un liquide inerte organique ; au moins un persulfate ; au moins un agent alcalin ; et l'autre contient une composition comprenant au moins un agent oxydant.

## Claims

1. Composition for bleaching human keratin fibres, **characterized in that** it comprises:
- at least one compound chosen from ethylenediamine-N,N'-disuccinic acid, one of its salts, or a mixture thereof,
- at least one inert organic liquid,
- at least one persulphate.

2. Composition according to the preceding claim, **characterized in that** the content of compound is between 0.01% and 20% by weight, relative to the weight of the composition, more particularly between 0.1% and 10% by weight, relative to the weight of the composition, preferably between 0.2% and 5% by weight, relative to the weight of the composition.

3. Composition according to either of the preceding claims, **characterized in that** the inert organic liquid comprises at least one compound chosen from polydecenes, carboxylic acid monoesters and polyesters, sugar monoesters or polyesters of C₈-C₃₀ acids, cyclic ethers, cyclic esters, silicone oils, mineral oils and plant oils, or mixtures thereof.

4. Composition according to one of the preceding claims, **characterized in that** the inert liquid is chosen from esters of C₈-C₃₀ acids and of a saturated, linear or branched C₃-C₆ monoalcohol.

5. Composition according to any one of the preceding claims, **characterized in that** the content of inert liquid represents from 0.5% to 35% by weight, relative to the weight of the composition.

6. Composition according to any one of the preceding claims, **characterized in that** the bleaching composition is in the form of a paste.

7. Composition according to the preceding claim, **characterized in that** the content of inert liquid represents from 15% to 35% by weight, relative to the weight of the composition.

8. Composition according to any one of the preceding claims, **characterized in that** the bleaching composition is in the form of a divided solid.

9. Composition according to the preceding claim, **characterized in that** the content of inert liquid represents from 0.5% to less than 15% by weight, relative to the weight of the composition.

10. Composition according to any one of the preceding claims, **characterized in that** the persulphate is chosen from sodium persulphates and potassium persulphates, alone or as a mixture.

11. Composition according to any one of the preceding claims, **characterized in that** the content of persulphate is between 10% and 70% by weight, relative to the weight of the composition, preferably between 20% and 60% by weight, relative to the weight of the composition.

12. Composition according to any one of the preceding claims, **characterized in that** it comprises at least one alkaline agent.

13. Composition according to the preceding claim, **characterized in that** the alkaline agent is chosen from urea; ammonium salts; and silicates, phosphates or carbonates of alkali metals or alkaline earth metals, alone or as a mixture.

14. Composition according to either of Claims 12 and 13, **characterized in that** the content of alkaline agent is between 0.01% and 40% by weight, relative to the weight of the composition, preferably between 0.1% and 30% by weight, relative to the weight of the composition.

15. Composition according to one of the preceding claims, **characterized in that** the composition contains at least one thickening polymer agent comprising or not comprising a hydrophobic chain.

16. Composition according to Claim 15, **characterized in that** the content of thickening agent represents 0.01% to 30% by weight, relative to the weight of the composition.

17. Composition according to one of the preceding claims, **characterized in that** the composition comprises less than 1% by weight of water, and preferably less than 0.5% by weight of water.

18. Ready-for-use bleaching composition which can be obtained by mixing the bleaching composition according to any one of the preceding claims with a composition comprising at least one oxidizing agent.

19. Ready-for-use composition according to the preceding claim, **characterized in that** the oxidizing agent is hydrogen peroxide.

20. Ready-for-use composition according to the preceding claim, **characterized in that** the content of hydrogen peroxide in the ready-for-use composition represents from 1% to 12% in the capacity of hydrogen peroxide, more particularly from 2% to 9% in the capacity of hydrogen peroxide, preferably from 2% to 6% in the capacity of hydrogen peroxide.

21. Ready-for-use composition according to any one of Claims 18 to 20, **characterized in that** the content of compound chosen from ethylenediamine-N,N'-disuccinic acid, salt or mixture represents from 0.05% to 10% by weight, relative to the weight of this composition, advantageously from 0.5% to 5% by weight, and preferably from 0.1% to 2.5% by weight, relative to the weight of the ready-for-use composition.

22. Ready-for-use composition according to any one of Claims 19 to 21, **characterized in that** the content of persulphate is between 5% and 35% by weight, relative to the weight of the ready-for-use composition, preferably between 10% and 30% by weight, relative to the weight of the ready-for-use composition.

23. Ready-for-use composition according to any one of Claims 19 to 22, **characterized in that** the content of alkaline agent is between 0.005% and 20% by weight, relative to the weight of the ready-for-use composition, preferably between 0.05% and 15% by weight, relative to the weight of the ready-for-use composition.

24. Ready-for-use composition according to any one of Claims 19 to 23, **characterized in that** it comprises at least one nonionic, anionic, amphoteric, zwitterionic or cationic surfactant, or mixtures thereof.

25. Ready-for-use composition according to either of Claims 19 and 24, **characterized in that** the total content of surfactant represents between 0.05% and 30% by weight, relative to the weight of the ready-for-use composition, preferably between 0.1% and 20% by weight, relative to the weight of the ready-for-use composition.

26. Ready-for-use composition according to one of Claims 19 to 25, **characterized in that** the pH of the ready-for-use composition is between 4 and 12, more particularly between 7 and 11.5, preferably between 8 and 11.

27. Process for bleaching human keratin fibres, in particular the hair, consisting in applying the ready-for-use composition according to any one of Claims 18 to 26, to the wet or dry human keratin fibres to be bleached; in leaving it to act for a leave-in time sufficient to obtain the desired bleaching; and in removing the composition by rinsing with water.

28. Process according to the preceding claim, **characterized in that,** after the rinsing with water, the treated fibres are washed with a shampoo, and then rinsed with water and, optionally, dried.

29. Process according to either of Claims 27 and 28, **characterized in that** the ready-for-use composition is prepared, before application thereof to the fibres, by mixing the bleaching composition according to any one of Claims 1 to 17 with a composition comprising at least one oxidizing agent.

30. Multicompartment device for carrying out the process according to one of Claims 27 to 29, **characterized in that** it comprises at least two compartments, one of which contains a bleaching composition comprising at least one compound chosen from ethylenediamine-N,N'-disuccinic acid, one of its salts, or a mixture thereof; at least one inert organic liquid; at least one persulphate and at least one alkaline agent; and the other of which contains a composition comprising at least one oxidizing agent.

## Patentansprüche

1. Zusammensetzung zum Entfärben von menschlichen Keratinfasern, **dadurch gekennzeichnet, dass** sie enthält:
• mindestens eine Verbindung, die unter Ethylendiamin-N,N'-dibernsteinsäure, einem ihrer Salze oder deren Gemischen ausgewählt ist,
• mindestens eine inerte organische Flüssigkeit,
• mindestens ein Persulfat.

2. Zusammensetzung nach dem vorhergehenden Anspruch, **dadurch gekennzeichnet, dass** der Mengenanteil der Verbindung im Bereich von 0,01 bis 20 Gew.-%, bezogen auf das Gesamtgewicht der Zusammensetzung, insbesondere im Bereich von 0,1 bis 10 Gew.-%, bezogen auf das Gesamtgewicht der Zusammensetzung, und vorzugsweise im Bereich von 0,2 bis 5 Gew.-%, bezogen auf das Gesamtgewicht der Zusammensetzung, liegt.

3. Zusammensetzung nach einem der vorhergehenden Ansprüche, **dadurch gekennzeichnet, dass** die inerte organische Flüssigkeit mindestens eine Verbindung enthält, die unter den Polydecenen, Mono- und Polyestern von Carbonsäuren, Mono- oder Polyestern von Zuckersäuren mit 8 bis 30 Kohlenstoffatomen, cyclischen Ethern, cyclischen Estern, Siliconölen, Mineralölen, pflanzlichen Ölen oder deren Gemischen ausgewählt ist.

4. Zusammensetzung nach einem der vorhergehenden Ansprüche, **dadurch gekennzeichnet, dass** die inerte Flüssigkeit unter den Estern von C₈₋₃₀-Säuren und einem linearen oder verzweigten, gesättigten Monoalkohol mit 3 bis 6 Kohlenstoffatomen ausgewählt ist.

5. Zusammensetzung nach einem der vorhergehenden Ansprüche, **dadurch gekennzeichnet, dass** der Mengenanteil der inerten Flüssigkeit 0,5 bis 35 Gew.-%, bezogen auf das Gesamtgewicht der Zusammensetzung, ausmacht.

6. Zusammensetzung nach einem der vorhergehenden Ansprüche, **dadurch gekennzeichnet, dass** die Zusammensetzung zum Entfärben in der Form einer Paste vorliegt.

7. Zusammensetzung nach dem vorhergehenden Anspruch, **dadurch gekennzeichnet, dass** der Mengenanteil der inerten Flüssigkeit 15 bis 35 Gew.-%, bezogen auf das Gesamtgewicht der Zusammensetzung, ausmacht.

8. Zusammensetzung nach einem der vorhergehenden Ansprüche,
**dadurch gekennzeichnet, dass** die Zusammensetzung zum Entfärben in der Form eines verteilten Feststoffes vorliegt.

9. Zusammensetzung nach dem vorhergehenden Anspruch, **dadurch gekennzeichnet, dass** der Mengenanteil der inerten Flüssigkeit 0,5 bis weniger als 15 Gew.-%, bezogen auf das Gesamtgewicht der Zusammensetzung, ausmacht.

10. Zusammensetzung nach einem der vorhergehenden Ansprüche,
**dadurch gekennzeichnet, dass** das Persulfat einzeln oder in Form von Gemischen unter den Persulfaten von Natrium oder Kalium ausgewählt ist.

11. Zusammensetzung nach einem der vorhergehenden Ansprüche, **dadurch gekennzeichnet, dass** der Mengenanteil des Persulfats im Bereich von 10 bis 70 Gew.-%, bezogen auf das Gesamtgewicht der Zusammensetzung, und vorzugsweise im Bereich von 20 bis 60 Gew.-%, bezogen auf das Gesamtgewicht der Zusammensetzung, liegt.

12. Zusammensetzung nach einem der vorhergehenden Ansprüche, **dadurch gekennzeichnet, dass** sie mindestens ein Alkalisierungsmittel enthält.

13. Zusammensetzung nach dem vorhergehenden Anspruch, **dadurch gekennzeichnet, dass** das Alkalisierungsmittel einzeln oder in Form von Gemischen unter Harnstoff; Ammoniumsalzen; Silicaten, Phosphaten, Carbonaten von Alkalimetallen oder Erdalkalimetallen ausgewählt ist.

14. Zusammensetzung nach einem der Ansprüche 12 oder 13, **dadurch gekennzeichnet, dass** der Mengenanteil des Alkalisierungsmittels im Bereich von 0,01 bis 40 Gew.-%, bezogen auf das Gesamtgewicht der Zusammensetzung, und vorzugsweise im Bereich von 0,1 bis 30 Gew.-%, bezogen auf das Gesamtgewicht der Zusammensetzung, liegt.

15. Zusammensetzung nach einem der vorhergehenden Ansprüche, **dadurch gekennzeichnet, dass** die Zusammensetzung mindestens ein polymeres Verdickungsmittel enthält, das gegebenenfalls eine hydrophobe Kette aufweist.

16. Zusammensetzung nach Anspruch 15, **dadurch gekennzeichnet, dass** der Mengenanteil des Verdickungsmittels 0,01 bis 30 Gew.-%, bezogen auf das Gesamtgewicht der Zusammensetzung, ausmacht.

17. Zusammensetzung nach einem der vorhergehenden Ansprüche, **dadurch gekennzeichnet, dass** die Zusammensetzung weniger als 1 Gew.-% Wasser und vorzugsweise weniger als 0,5 Gew.-% Wasser enthält.

18. Gebrauchsfertige Zusammensetzung zum Entfärben, die durch Mischen der Zusammensetzung zum Entfärben nach einem der vorhergehenden Ansprüche mit einer Zusammensetzung, die mindestens ein Oxidationsmittel enthält, erhältlich ist.

19. Gebrauchsfertige Zusammensetzung nach dem vorhergehenden Anspruch, **dadurch gekennzeichnet, dass** es sich bei dem Oxidationsmittel um Wasserstoffperoxid handelt.

20. Gebrauchsfertige Zusammensetzung nach dem vorhergehenden Anspruch, **dadurch gekennzeichnet, dass** der Mengenanteil des Wasserstoffperoxids in der gebrauchsfertigen Zusammensetzung 1 bis 12 %, ausgedrückt als Titer Wasserstoffperoxid, insbesondere 2 bis 9 %, ausgedrückt als Titer Wasserstoffperoxid, und vorzugsweise 2 bis 6 %, ausgedrückt als Titer Wasserstoffperoxid, ausmacht.

21. Gebrauchsfertige Zusammensetzung nach einem der Ansprüche 18 bis 20, **dadurch gekennzeichnet, dass** der Mengenanteil der Verbindung, die unter Ethylendiamin-N,N'-dibernsteinsäure, ihren Salzen oder Gemischen ausgewählt ist, 0,05 bis 10 Gew.-%, bezogen auf das Gesamtgewicht der Zusammensetzung, vorteilhaft 0,5 bis 5 Gew.-% und vorzugsweise 0,1 bis 2,5 Gew.-%, bezogen auf das Gesamtgewicht der gebrauchsfertigen Zusammensetzung, ausmacht.

22. Gebrauchsfertige Zusammensetzung nach einem der Ansprüche 19 bis 21, **dadurch gekennzeichnet, dass** der Mengenanteil des Persulfats im Bereich von 5 bis 35 Gew.-%, bezogen auf das Gesamtgewicht der gebrauchsfertigen Zusammensetzung, und vorzugsweise im Bereich von 10 bis 30 Gew.-%, bezogen auf das Gesamtgewicht der gebrauchsfertigen Zusammensetzung, liegt.

23. Gebrauchsfertige Zusammensetzung nach einem der Ansprüche 19 bis 22, **dadurch gekennzeichnet, dass** der Mengenanteil des Alkalisierungsmittels im Bereich von 0,005 bis 20 Gew.-%, bezogen auf das Gesamtgewicht der gebrauchsfertigen Zusammensetzung, und vorzugsweise im Bereich von 0,05 bis 15 Gew.-%, bezogen auf das Gesamtgewicht der gebrauchsfertigen Zusammensetzung, liegt.

24. Gebrauchsfertige Zusammensetzung nach einem der Ansprüche 19 bis 23, **dadurch gekennzeichnet, dass** sie mindestens einen nichtionischen, anionischen, amphoteren, zwitterionischen oder kationischen grenzflächenaktiven Stoff oder deren Gemische enthält.

25. Gebrauchsfertige Zusammensetzung nach einem der Ansprüche 19 oder 24, **dadurch gekennzeichnet, dass** die Gesamtmenge an grenzflächenaktivem Stoff 0,05 bis 30 Gew.-%, bezogen auf das Gesamtgewicht der gebrauchsfertigen Zusammensetzung, und vorzugsweise 0,1 bis 20 Gew.-%, bezogen auf das Gesamtgewicht der gebrauchsfertigen Zusammensetzung, ausmacht.

26. Gebrauchsfertige Zusammensetzung nach einem der Ansprüche 19 bis 25, **dadurch gekennzeichnet, dass** der pH-Wert der gebrauchsfertigen Zusammensetzung im Bereich von 4 bis 12, insbesondere 7 bis 11,5 und vorzugsweise 8 bis 11 liegt.

27. Verfahren zum Entfärben von menschlichen Keratinfasern und insbesondere Haaren, das darin besteht, die gebrauchsfertige Zusammensetzung nach einem der Ansprüche 18 bis 26 auf die trockenen oder feuchten, zu bleichenden menschlichen Keratinfasern aufzutragen; sie während einer Zeitspanne, die ausreichend ist, um die gewünschte Entfärbung zu erreichen, einwirken zu lassen; und die Zusammensetzung durch Spülen mit Wasser zu entfernen.

28. Verfahren nach dem vorhergehenden Anspruch, **dadurch gekennzeichnet, dass** die behandelten Fasern nach dem Spülen mit einem Haarwaschmittel gewaschen werden und dann mit Wasser gespült und gegebenenfalls getrocknet wird.

29. Verfahren nach einem der Ansprüche 27 oder 28, **dadurch gekennzeichnet, dass** die gebrauchsfertige Zusammensetzung vor dem Auftragen auf die Fasern hergestellt wird, indem die Zusammensetzung zum Entfärben nach einem der Ansprüche 1 bis 17 mit einer Zusammensetzung vermischt wird, die mindestens ein Oxidationsmittel enthält.

30. Vorrichtung mit mehreren Abteilungen für die Durchführung des Verfahrens nach einem der Ansprüche 27 bis 29, **dadurch gekennzeichnet, dass** sie mindestens zwei Abteilungen aufweist, wobei eine Abteilung eine Zusammensetzung zum Entfärben, die mindestens eine Verbindung, die unter Ethylendiamin-N,N'-dibernsteinsäure, einem ihrer Salze oder deren Gemischen ausgewählt ist; mindestens eine inerte organische Flüssigkeit; mindestens ein Persulfat; und mindestens einen alkalischen Stoff enthält; und die andere Abteilung eine Zusammensetzung mit mindestens einem Oxidationsmittel enthält.
